(19) Europäisches Patentamt
European Patent Office
Office européen des brevets

(11) Publication number: 0 323 147 A2

# (12) EUROPEAN PATENT APPLICATION

(21) Application number: 88312194.9

(51) Int. Cl.4: C07D 239/40 , A61K 31/505

(22) Date of filing: 22.12.88

Claims for the following Contracting States: ES + GR.

(30) Priority: 31.12.87 US 140052

(43) Date of publication of application: 05.07.89 Bulletin 89/27

(84) Designated Contracting States: AT BE CH DE ES FR GB GR IT LI LU NL SE

(71) Applicant: SMITHKLINE BECKMAN CORPORATION
One Franklin Plaza P O Box 7929
Philadelphia Pennsylvania 19103(US)

(72) Inventor: Kruse, Lawrence Ivan
33 Firs Walk
Tewin Hertfordshire AL6 0NY(GB)
Inventor: Ross, Stephen Torey
718 Old State Road
Berwyn Pennsylvania 19312(US)

(74) Representative: Waters, David Martin, Dr. et al
Smith Kline & French Laboratories Ltd.
Patent Department Mundells
Welwyn Garden City Hertfordshire AL7 1EY(GB)

## (54) 1-Aralkyl-1,2-dihydropyrimidine-2-thiones.

(57) Compounds of structure (I)

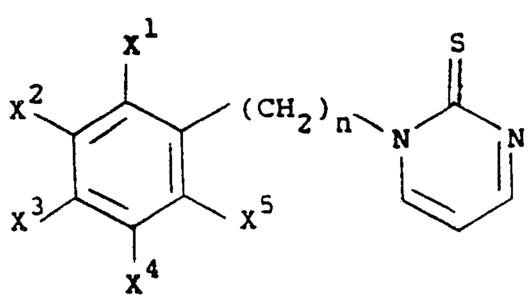

(I)

and pharmaceutically acceptable acid addition salts thereof are described in which, n is 0 to 5; and $X^1$ to $X^5$ are any accessible combination of hydrogen, halogen, $C_{1-6}$alkyl, $C_{1-6}$alkoxy, cyano, nitro, $SONH_2$, $SO_2CH_3$, $SO_2CH_2F$, $SO_2CHF_2$, $SO_2CF_3$, $CF_3$, CHO, OH, $CH_2OH$, $CO_2H$, $CO_2C_pH_{2p+1}$ wherein p is 1 to 4.

These compounds are dopamine-β-hydroxylase inhibitors. Pharmaceutical compositions are described as are methods of use. Processes for the preparation of these compounds are described.

EP 0 323 147 A2

## 1-Aralkyl-1,2-dihydropyrimidine-2-thiones

The present invention relates to novel substituted 1-aralkyl-1,2-dihydropyrimidine-2-thiones and acid addition salts thereof, processes for their preparation, intermediates useful in their preparation, pharmaceutical compositions containing them and their use as medicaments.

Compounds that inhibit DBH activity are well known in the art and include :

(a) 5-alkylpicolinic acids [See, Suda et al., Chem. Pharm. Bull. 17, 2377 (1969); Umezawa et al., Biochem. Pharmacol. 19, 35 (1969); Hidaka et al., Mol. Pharmacol. 9, 1972 (1973); Miyano et al., Chem. Pharm. Bull. 26, 2328 (1978); Miyano et al., Heterocycles 14, 755 (1980); Claxton et al., Eur. J. Pharmacol. 37, 179 (1976)];

(b) BRL 8242 [See, Claxton et al., Eur. J. Pharmacol. 37, 179 (1976)];

(c) 1-alkylimidazole-2-thiols [See, Hanlon et al., Life Sci. 12, 417 (1973); Fuller et al., Adv. Enzyme Regul. 15 267 (1976)];

(d) substituted thioureas [See, Johnson et al., J. Pharmacol. Exp. Ther. 168, 229 (1969)]; and

(e) benzyloxamine and benzylhydrazine [See, Creveling et al., Biochim. Biophys Acta 64, 125 (1962); Creveling et al., Biochim. Biophys. Acta 8, 215 (1962); Van De Schoot et al., J. Pharmacol. Exp. Ther. 141, 74 (1963); Bloom, Ann N.Y. Acad. Sci. 107, 878 (1963)].

(f) Fusaric acid derivatives and analogues [See, Runti et al., Il Farmaco Ed. Sci. 36, 260 (1980)] for example phenylpicolinic acid, 5-(4-chlorobutyl) picolinic acid, substituted amides of fusaric acid and acids and amides of 5-butydropicolinic acid, 5-aminopicolinic acid, 5-hydrazinopicolinic acid, and derivatives thereof.

(g) Hidaka et al., Molecular Pharmacology, 9, 172-177 (1972) 5-(3,4-dibromobutyl)picolinic acid and5-(dimethyldithiocarbamoyl)methylpicolinic acid.

(h) Bupicomide, 5-(n-butyl)picolinamide, is reportedby Ehrreich et al., "New Antihypertensive Drugs", Spectrum Publications, 1976, pg. 409-432,

(i) In United States Patent No. 4,532,331 a series of 1-phenyl and 1-phenylalkylimidazole compounds having a mercapto or alkylthio group in the 2-position are disclosed.

(j) United States Patent No. 4,487,761 describes several methylpyridine derivatives isolated from the fermentation broth of a strain of Streptoverticillium.

(k) Friedman et al., Psychosomatic Med. 40, 107 (1978), report that patients treated with alpha-methyl- DOPA, guanethidine, and reserpine, but not propranolol and diuretics, have lowered DBH levels, although the significance of the observation is uncertain.

(l) In United States Patent No. 3,448,423 are disclosed compounds having the formula:

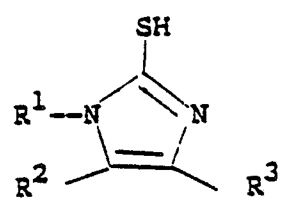

in which $R^2$ and $R^3$ can be H, and $R^1$ can be substituted phenyl. The compounds are said to have analgesic, anti- inflammatory and antipyretic properties. Gerbert et al., US patent 3,915,980, disclose such compounds wherein $R^1$ can be phenyl or phenyl($C_{1-3}$) alkyl, as intermediates to imidazolyl-2-thioalkanoic acid esters.

(m) Iverson, Acta Chem. Scan. 21, 279 (1967) reports compounds having the formula :

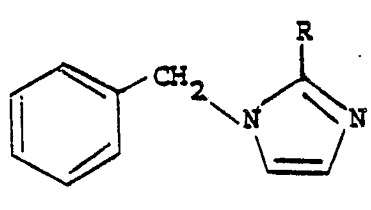

wherein R can be $-CO_2H$ or $-CH_2NHC_6H_5$, but does not report pharmaceutical uses for the compounds.

Non-specific, often toxic effects of known DBH inhibitors have obviated clinical use of these compounds. Fusaric acid, for example, is hepatotoxic. See, for example, Teresawa et al., Japan Cir. J. 35, 339 (1971) and references cited therein.

Therefore there is a continuing need for novel compounds that possess DBH inhibitory activity.

Accordingly the present invention provides compounds of structure (I) :

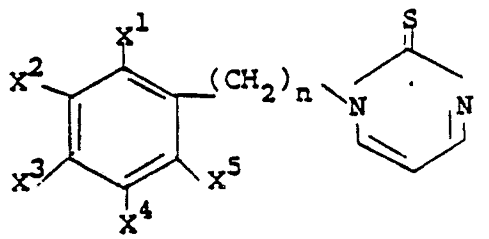

(I)

or pharmaceutically acceptable acid addition salts thereof in which,

n is 0 to 5; and

$X^1$ to $X^5$ are any accessible combination of hydrogen, halogen, $C_{1-6}$alkyl, $C_{1-6}$alkoxy, cyano, nitro, $SONH_2$, $SO_2CH_3$, $SO_2CH_2F$, $SO_2CHF_2$, $SO_2CF_3$, $CF_3$, CHO,OH, $CH_2OH$, $CO_2H$, or

$CO_2C_pH_{2p+1}$ wherein p is 1 to 4.

Suitably $X^1$ to $X^5$ are hydrogen. More suitably at least one of $X^1$ to $X^5$ is halogen and the others arehydrogen. Preferably, one or two of $X^1$ to $X^5$ are halogen. More preferably $X^2$ is halogen and $X^4$ is hydrogen or halogen. Most prefered are compounds in which $X^4$ is fluoro or those in which $X^4$ and $X^2$ are fluoro.

As used herein "accessible combination" means any combination of the substituents that is available by chemical synthesis and is stable.

Preferably n is 1; suitably n is 0 or 2 to 5;

Particular compounds of this invention include;

1-benzyl-1,2-dihydropyrimidine-2-thione,

1-(3-fluorobenzyl)-1,2-dihydropyrimidine-2-thione hydrochloride, and

1-(3,5-difluorobenzyl)-1,2-dihydropyrimidine 2-thione hydrochloride.

Suitable acid addition salts of the present invention include hydrochloride, hydrobromide, hydroiodide, trifluoro-ethanoate, nitrate, sulphate or others known to those skilled in the art. Preferred addition salts are in particular pharmaceutically acceptable acid addition salts such as hydrochlorides.

A further aspect of the present invention provides a process for the preparation of compounds of structure (I) and pharmaceutically acceptable acid addition salts thereof, which comprises reacting of a compound of structure (II)

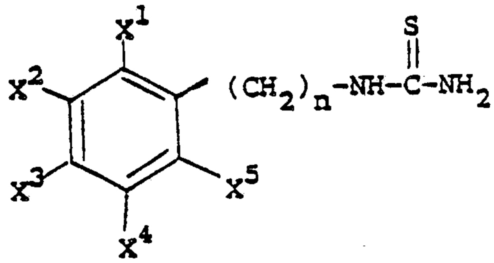

(II)

in which n is 0 to 5, and $X^1$ to $X^5$ are any accessible combination of hydrogen, halogen, $C_{1-6}$alkyl, $C_{1-6}$alkoxy, cyano, nitro, $SONH_2$, $SO_2CH_3$, $SO_2CH_2F$, $SO_2CHF_2$, $SO_2CF_3$, $CF_3$, CHO, $CH_2OC_{1-4}$alkyl or $CO_2C_{1-4}$alkyl with a compound of structure (III)

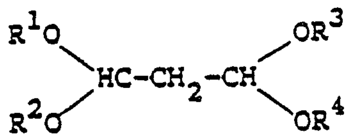

(III)

in which $R^1$ to $R^4$ are each $C_{1-6}$alkyl groups or $R^1$ and $R^2$ taken together and/or $R^3$ and $R^4$ taken together are ethylene and optionally thereafter converting a group $X^1$ to $X^5$ into a hydroxy, $CH_2OH$ or $CO_2H$ group and optionally forming a pharmaceutically acceptable acid addition salt thereof.

Suitably in structure (III) the $C_{1-6}$alkyl groups are in any combination. More suitably two of $R^1$ to $R^4$ are the same. Most suitably three of $R^1$ to $R^4$ are the same. Preferably all four of $R^1$ to $R^4$ are the same. More preferably $R^1$ to $R^4$ are all ethyl. Most preferably $R^1$ to $R^4$ are all methyl.

Suitably the reaction is carried out in an aqueous solvent in the presence of an acid. Suitable acids include for example hydrochloric acid, hydrobromic acid or sulphuric acid, preferably hydrochloric acid. Suitable aqueous solvents include aqueous lower alcohols such as $C_{1-6}$alcohols, suitably methanol or propanol; preferably ethanol or methanol; alternatively, a suitable inert aqueous solvent such as for example tetrahydrofuran or dioxane may be used. Preferably the reaction is carried out in aqueous ethanol in the presence of hydrochloric acid.

Compounds of structure (II) are either known in the literature or are easily prepared by the method of Neville and McGee, Can. J. Chem. (1963), 41, 2123. An example of such a compound is N-(3-fluorobenzyl)-thiourea. The compound of structure (III) which is malondialdehyde tetramethyl acetal is commercially available, other compounds of structure (III) can be prepared by methods known to those skilled in the art.

An alternative process for preparation of compounds of structure (I) and pharmaceutically acceptable addition salts thereof, comprises reacting a compound of structure (IV) :

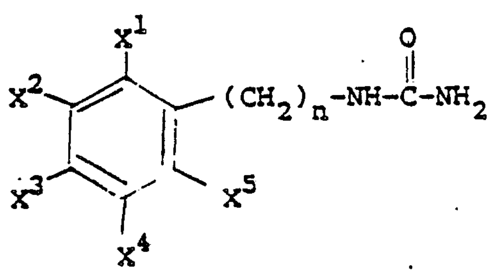

(IV)

in which n, $X^1$ to $X^5$ are as defined for compounds of the structure (II) with a compound of structure (III) as defined above in the presence of an acid to form a compound of structure (V):

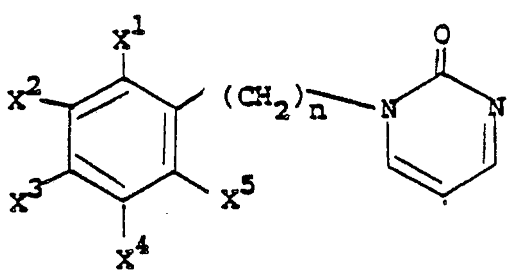

(V)

in which n and $X^1$ to $X^5$ are as defined for compounds of structure (II) followed by reaction with a compound of structure (VI) (Lawesson's reagent) :

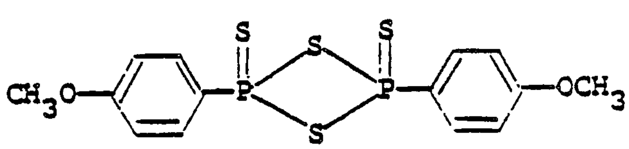

(VI)

to form compounds of structure (I) in which n and $X^1$ to $X^5$ are as defined for structure (II) and optionally thereafter converting a group $X^1$ to $X^5$ into a hydroxy, $CH_2OH$ or $CO_2H$ group and optionally forming a

pharmaceutically acceptable acid addition salt thereof.

Pharmaceutically acceptable acid addition salts of compounds of the invention are formed with appropriate strong or moderately strong organic or inorganic acids by methods known in the art. For example, the base is reacted with a suitable inorganic or organic acid in an aqueous miscible solvent such as ethanol with isolation of the salt by removing the solvent or in an aqueous immiscible solvent when the acid is soluble therein, such as ethyl ether or chloroform, with the desired salt separating directly or isolated by removing the solvent. Exemplary of the salts which are included in this invention include methanesulfonate, ethanesulfonate, benzenesulfonate, hydrochloride, hydrobromide, sulfate, trifluoroacetate, phosphate, and nitrate salts.

The present invention also provides a compound of structure (I) or a pharmaceutically acceptable salt for use as a medicament, for example, for use as a dopamine-$\beta$-hydroxylase inhibitor.

Because the compounds of structure (I) inhibit DBH activity they have therapeutic value as diuretic, natriuretic, cardiotonic, antihypertensive, and vasodilatoragents, as well as antiulcerogenic and anti-Parkinson agents. Listed in Table I are the compounds of the invention that were tested for in vitro DBH inhibition by a standard procedure for assaying conversion of tyramine to octopamine in the presence of DBH. J.J. Pisano, et al., Biochim. Biophys. Acta, 43, 566-568 (1960). Octopamine was assayed following sodium periodate oxidation to p-hydroxy-benzaldehyde by measuring spectrophotometric absorbance at 330 nm. In Table I, inhibition is given in micromolar concentration of compound at which DBH activity was halved ($IC_{50}$). By this test, fusaric acid had an $IC_{50}$ of 0.8 micromolar.

Table I

| Compound | DBH $IC_{50}$ (μM) |
|---|---|
| 1-(3-fluorobenzyl)-1,2,dihydropyrimidine-2-thione hydrochloride | 15%[a] |
| 1-(3,5-difluorobenzyl)-1,2,dihydropyrimidine-2-thione hydrochloride | 2.8 |
| 1-benzyl-1,2-dihydropyrimidine-2-thione | 5%[a] |

a-percent inhibition at $10^{-5}$ M compound concentration. $IC_{50}$ not determined.

Further, spontaneously hypertensive rats were treated with a suspension or solution of 1-(3,5-fluorobenzyl)-1,2,-dihydropyrimidine-2-thione hydrochloride at a dose of 50 mg/kg orally and mean arterial blood pressure was monitored for 260 minutes using indwelling cannulae in the tail arteries. When compared to vehicle-treated controls, animals treated with the compounds of the invention exhibited significant blood pressure reductions within approximately 30 minutes after treatment. Maximal blood pressure reduction was approximately 25 to 30 mm Hg.

The compounds of structure (I) usually are administered in a standard pharmaceutical composition. The present invention therefore provides in a further aspect pharmaceutical compositions comprising a compound of structure (I) or a pharmaceutically acceptable salt thereof and a pharmaceutically acceptable carrier. Such compositions include those suitable for administration by an appropriate route known to those skilled in the art for example, orally, parenterally, transdermally, rectally, by inhalation or by buccal administration.

The compounds of structure (I) and their pharmaceutically acceptable salts which are active when given orally can be formulated as liquids, for example syrups, suspensions or emulsions, tablets, capsules and lozenges.

A liquid formulation generally will consist of a suspension or solution of the compound or a pharmaceutically acceptable salt in a suitable liquid carrier(s) for example, ethanol, glycerine, sorbitol, non-aqueous solvent (for example polyethylene glycol) oils, or water with a suspending agent, preservative surfactant, wetting agent, flavouring or colouring agent.

Alternatively, a liquid formulation is prepared from a reconstitutable powder. For example a powder containing active compound, suspending agent, sucrose and a sweetener is reconstituted with water to form a suspension; and a syrup is prepared from a powder containing active ingredient, sucrose and a sweetener.

A composition in the form of a tablet is prepared using any suitable pharmaceutical carrier(s) routinely used for preparing solid formulations. Examples of such carriers include magnesium stearate, starch, lactose, sucrose, cellulose and binders, for example, polyvinyl pyrrolidone. The tablet also is provided with a color film coating, or color is included as part of the carrier(s). In addition, acting compound is formulated

in a controlled release dosage form as, for example, a tablet comprising a hydrophilic or hydrophobic matrix.

A composition in the form of a capsule is preparedusing routine encapsulation procedures. For example,. pellets containing the active ingredient are prepared using standard carriers and then filled into a hard gelatin capsule; alternatively, a dispersion or suspension is prepared using any suitable pharmaceutical carrier(s), for example aqueous gums, celluloses, silicates or oils and the dispersion or suspension then filled into a soft gelatin capsule.

Typical parenteral compositions consist of a solution or suspension of the compound or pharmaceutically acceptable salt in a sterile aqueous carrier or parenterally acceptable oil, for example polyethylene glycol, polyvinyl pyrrolidone, lecithin, arachis oil or sesame oil. Alternatively, the solution can be lyophilised and then reconstituted with a suitable solvent just prior to administration.

A typical suppository formulation comprises a compoundof structure (I) or a pharmaceutically acceptable salt thereof which is active when administered in this way, with a binding and/or lubricating agent such as polymeric glycols, gelatins or cocoa butter or other low melting vegetable or synthetic waxes or fats.

Compounds of structure (I) and their pharmaceutically acceptable addition salts which are active on topical administration can be formulated as transdermal compositions. Such compositions include, for example, a backing, active compound reservoir, a control membrane, liner and contact adhesive.

Typical compositions for inhalation are in the form of a solution, suspension or emulsion that may be administered in the form of an aerosol using a conventional propellant such a dichlorodifluoromethane or trichlorofluoromethane.

Preferably the composition is in an appropriate unit dosage form. Each dosage unit for oral administration contains preferably from 1 to 250 mg (and for parenteral administration contains preferably from 0.1 to 25 mg) of a compound of the structure (I) or a pharmaceutically acceptable salt thereof.

The daily dosage regimen for an adult patient may be, for example, an oral dose of between 1 mg and 1000 mg, preferably between 1 mg and 250 mg, or an intravenous, subcutaneous, or intramuscular dose of between 0.1 mg and 100 mg, preferably between 0.1 mg and 25 mg, of the compound of the structure (I) or a pharmaceutically acceptable salt thereof , the compound being administered 1 to 4 times per day. Suitably the compounds are administered for a period of continuous therapy, for example for a week or more. In addition, the compounds of this invention may be co-administered with other pharmaceutically active compounds, for example in combination, concurrently or sequentially.

The following Examples illustrate the invention. Temperatures are recorded in degrees centigrade.

## Example 1

### 1-Benzyl-1,2-dihydropyrimidine-2 thione

An 8.0 g (0.0482 mole) quantity of N-benzylthiourea. (prepared by the method of Neville and McGee, Can.J.Chem. (1963), 41, 2123) in 51% yield, m.p. 162-164° C and 7.91 g (0.0482 mole) of malondialdehyde tetramethyl acetal (Aldrich) were stirred in a mixture of 16 ml 12N hydrochloric acid, 65 ml water and 65 ml ethanol and the mixture heated at reflux (85° C) for 25 minutes. A clear solution formed at 55° C. Cooling caused the separation of a brown oil. The mixture was stirred with ether and the ether/aqueous solution decanted from the insoluble oil which was discarded.

The ether phase was separated and discarded and the aqueous phase was neutralized with aqueous sodium hydroxide which separated an oil. This oil was taken up in chloroform and the aqueous phase re-extracted twice with chloroform. Concentration of the combined extracts gave 6.2 g of an oil which slowly crystallized. This oil was taken up with ethanol which yielded 1.9 g of crystalline solid, m.p. 137.5-139° C. A small sample was recrystallized again from ethanol, m.p. 140-141° C. (Anal. Calcd. for $C_{11}H_{10}N_2S$: C, 65.32; H, 4.98; N, 13.85; S, 15.85.
Found: C, 65,71; H, 5.09; N, 13.95; S, 16.15).

## Example 2

1-(3-Fluorobenzyl)-1,2-dihydropyrimidine 2-thione Hydrochloride

A 9.5 g (0.0516 mole) quantity of N-(3-fluorobenzyl)-thiourea (prepared by the method of Neville and McGee (see Example 1) in 51% yield, m.p. 140-141 °C) and 8.5 g (0.0516 mole) of malondialdehyde tetramethyl acetal (Aldrich) were stirred in a mixture of 100 ml water, 100 ml methanol and 20 ml 12N hydrochloric acid for 18 hours at ambient temperature. The insoluble solid which had formed was filtered and discarded. The filtrate was refluxed for one hour, then cooled and concentrated to give a mixture of oil and crystalline solid. This solid was triturated with methanol to give 1.7 g, m.p. 173-175 °C dec. This was recrystallized from methanol to give 0.86 g, m.p. 182-183 °C dec.
Anal. Calcd. for $C_{11}H_9FN_2S.HCl$: C, 51.46; H, 3.93; N, 10.91.
Found: C, 51.40; H, 4.09; N,10.88.

Example 3

1-(3,5-Difluorobenzyl)-1,2-dihydropyrimidine 2-thione Hydrochloride

A 2.0 g (9.9 mmole) quantity of N-(3,5-difluorobenzyl)thiourea (prepared by the method of Neville and McGee (see Example 1) in 62% yield, m.p. 124-125 °C) and 1.63 g (9.9 mmole) of malondialdehyde tetramethyl acetal were dissolved in 40 ml methanol containing 2 ml of 12N hydrochloric acid. The solution was stirred overnight at ambient temperature and then refluxed for one hour. Concentration of the solution gave a moist yellow solid. This solid was taken up in methanol and ethyl acetate was added to give three crops of crystalline solid. Thin-layer chromatrography using silica gel GF plates and 10% methanol in chloroform as mobile phase indicated crops 1 and 3 primarily were desired product (Rf. 0.6), total 1.52 g. An attempt to further purify this combined material by recrystallization from boiling methanol led to partial decomposition. Concentration of the methanol solution gave a solid which was triturated with acetonitrile to give 0.5 g of crude product. This product was converted to the free base by neutralization of an aqueous methanolic solution with sodium carbonate and extraction with methylene chloride. Concentration of the combined methylene chloride extracts gave 330 mg of an oil which was chromatographed on a column of 50 g of "flash" silica gel using 5% methanol in chloroform. This yielded a total of 270 mg on concentration. The oil was taken up in methylene chloride and a slight excess of ethereal hydrogen chloride added to precipitate the hydrochloride salt, m.p. 183-184° dec.
Anal. Calcd. for $C_{11}H_8F_2N_2S.HCl$; C, 48.09; H, 3.30; N, 10.20; S, 11. 67.
Found: C,48.00; H. 3.42; N, 10.17: S,11.68.

Example 4

1-(3-Ethoxybenzyl)-1,2-dihydropyrimidine-2 thione

The process of example 1 in which N-benzylthiourea is replaced by 3-ethoxybenzylthiourea yields 1-(3-ethoxybenzyl)-1,2-dihydropyrimidine-2-thione.

Example 5

1-(2,3,5,6-tetrafluorobenzyl)-1,2dihydropyrimidine 2-thione

The process of example 1 in which N-benzylurea is replaced by 2,3,5,6-tetrafluorobenzylurea yields 1-(2,3,5,6-tetrafluorobenzyl)1,2-dihydropyrimidine-2-thione.

Example 6

1-(2,4-Dimethoxy 3,5-dichlorobenzyl)-1,2-dihydropyrimidine-2 thione

The process of example 1 in which N-benzylthiourea is replaced by 2,4-dimethoxy-3,5-dichlorobenzylthiourea yields 1-(2,4-dimethoxy-3,5-dichlorobenzyl)-1,2-dihydropyrimidine-2-thione.

Example 7

1-Phenylpropyl-1,2-dihydropyrimidine-2-thione

The process of example 1 in which N-benzylamine is replaced by N-phenylpropylamine yields 1-phenylpropyl-1,2-dihydropyrimidine-2-thione.

Example 8

An oral dosage form for administering the presently invented compounds is produced by screening, mixing, and filling into hard gelatin capsules the ingredients in the proportions shown in Table II, below.

Table II

| Ingredients | Amounts |
|---|---|
| 1-(3,5-Difluorobenzyl)-1,2-dihydropyrimidine 2-thione hydrochloride | 50 mg |
| magnesium stearate | 5 mg |
| lactose | 75 mg |

Example 9

The sucrose, calcium sulfate dihydrate, and structure(I) compound shown in Table III below, are mixed and granulated in the proportions shown with a 10% gelatin solution. The wet granules are screened, dried, mixed with the starch, talc and stearic acid, screened and compressed into a tablet.

Table III

| Ingredients | Amounts |
|---|---|
| 1-(3,5-Difluorobenzyl)-1,2-dihydropyrimidine 2-thione hydrochloride | 100 mg |
| calcium sulfate dihydrate | 150 mg |
| sucrose | 20 mg |
| starch | 10 mg |
| talc | 5 mg |
| stearic acid | 3 mg |

Example 10

1-(3,5-Difluorobenzyl)-1,2-dihydro-pyrimidine 2-thior hydrochloride,75 mg, is dispersed in 25 ml of normal saline to prepare an injectable preparation.

Contemplated equivalents of compounds of structure(I) are compounds that upon administration to mammals, including humans, are metabolized to compounds of structure(I) or metabolized to any active metabolites of compounds of structure(I) at a sufficient rate and in sufficient amounts to produce the physiological activity of compounds of structure(I). Such compounds also are included in the invented pharmaceutical compositions and used in the invented methods.

While the preferred embodiments of the invention are illustrated by the above, the invention is not limited to the precise instructions herein disclosed and the right to all modifications coming within the scope of the following claims is reserved.

## Claims

1. A compound of formula (I):

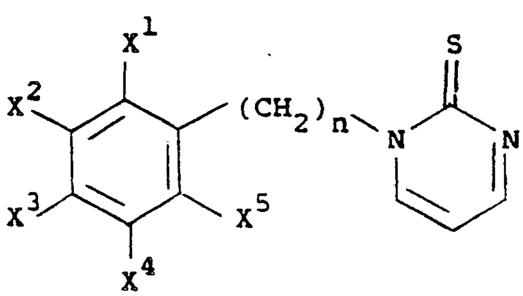

(I)

in which:
n is 0 to 5; and
$X^1$ to X5 are any accessible combination of hydrogen, halogen, $C_{1-6}$alkyl, $C_{1-6}$alkoxy, cyano, nitro, $SONH_2$, $SO_2CH_3$, $SO_2CH_2F$, $SO_2CHF_2$, $SO_2CF_3$, $CF_3$, CHO, OH, $CH_2OH$, $CO_2H$, $CO_2C_pH_{2p+1}$ wherein p is 1 to 4;
or a pharmaceutically acceptable acid addition salt thereof.

2. A compound of claim 1 in which n is 1.

3. A compound according to claim 1 or 2 in which one or two of $X^1$ to $X^5$ is halogen.

4. A compound according to claim 1 or 2 in which $X^4$ is halogen or $X^4$ and $X^2$ are halogen.

5. A compound according to claim 1 which is:
1(3-fluorobenzyl)-1,2-dihydropyrimidine-2-thione;
1-(3,5-difluorobenzyl)-1-2-dihydropyrimidine-2-thione; or
1 benzyl-1-2 dihydropyrimidine-2-thione;
or a pharmaceutically acceptable acid addition salt thereof.

6. A pharmaceutical composition comprising a compound according to any one of claims 1 to 5 and a pharmaceutically acceptable carrier.

7. A compound according to any one of claims 1 to 5 for use as a medicament.

8. A compound according to any one of claims 1 to 5 for use as a dopamine-$\beta$-hydroxylase inhibitor.

9. A process for the preparation of a compound of structure (I) or a pharmaceutically acceptable acid addition salt thereof as defined in claim 1, which comprises :

a) reacting a compound of formula (II):

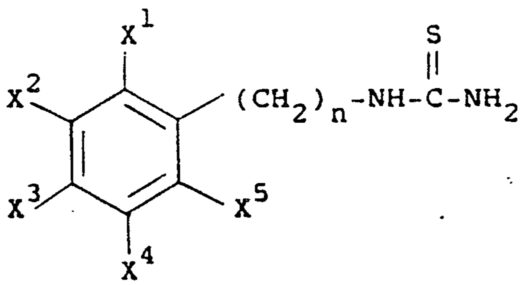

(II)

in which n is 0 to 5, and $X^1$ to $X^5$ are any accessible combination of hydrogen, halogen, $C_{1-6}$alkyl, $C_{1-6}$alkoxy, cyano, nitro, $SONH_2$, $SO_2CH_3$, $SO_2CH_2F$, $SO_2CHF_2$, $SO_2CF_3$, $CF_3$, CHO, $CH_2OC_{1-4}$alkyl or $CO_2C_{1-4}$alkyl with a compound of formula (III):

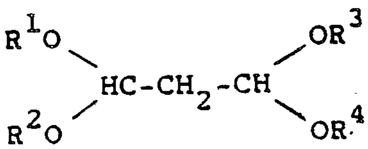

(III)

in which $R^1$ to $R^4$ are each $C_{1-6}$alkyl groups or $R^1$ and $R^2$ taken together and/or $R^3$ and $R^4$ taken together are ethylene; or

b) reacting a compound of formula (IV):

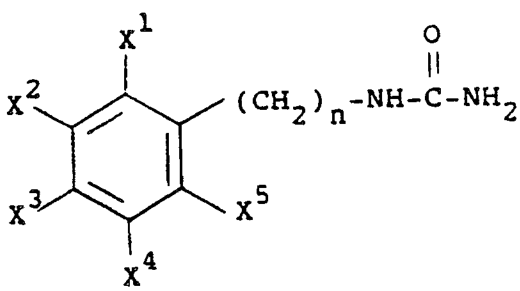

(IV)

in which n, $X^1$ to $X^5$ are as defined for compounds of the formula (II)

(1) with a compound of formula (III) as defined above in the presence of an acid to form a compound of formula (V):

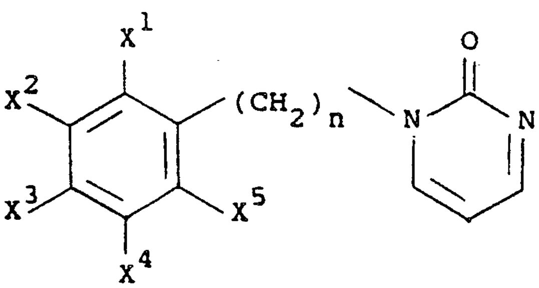

(V)

in which n and $X^1$ to X5 are as defined for compounds of formula (II)

(2) followed by reaction with a compound of formula (VI) (Lawesson's reagent) :

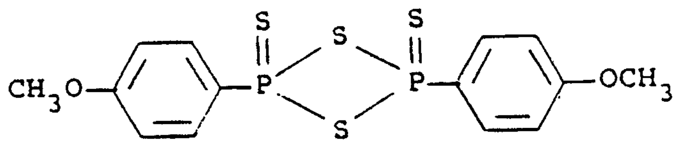

(VI)

to form a compound of formula (I) in which n and $X^1$ to $X^5$ are as defined for formula (II); and optionally thereafter:

(1) converting a group $X^1$ to $X^5$ into a hydroxy, $CH_2OH$ or $CO_2H$ group and

(2) forming a pharmaceutically acceptable acid addition salt thereof.

Claims for the following Contracting States : ES, GR

1. A process for preparing a compound of formula (I) :

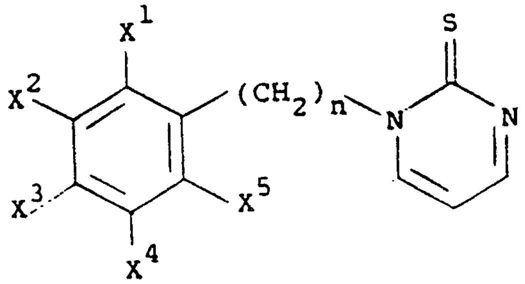

(I)

in which:

n is 0 to 5; and

$X^1$ to $X^5$ are any accessible combination of hydrogen, halogen, $C_{1-6}$alkyl, $C_{1-6}$alkoxy, cyano, nitro, $SONH_2$, $SO_2CH_3$, $SO_2CH_2F$, $SO_2CHF_2$, $SO_2CF_3$, $CF_3$, CHO, OH, $CH_2OH$, $CO_2H$, $CO_2C_pH_{2p+1}$ wherein p is 1 to 4;

or a pharmaceutically acceptable acid addition salt thereof,

which process comprises :

a) reacting a compound of formula (II):

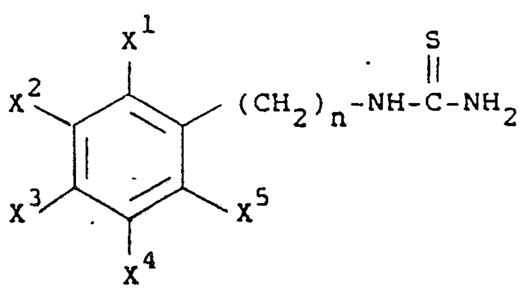

(II)

in which n is 0 to 5, and $X^1$ to $X^5$ are any accessible combination of hydrogen, halogen, $C_{1-6}$alkyl, $C_{1-6}$-alkoxy, cyano, nitro, $SONH_2$, $SO_2CH_3$, $SO_2CH_2F$, $SO_2CHF_2$, $SO_2CF_3$, $CF_3$, CHO, $CH_2OC_{1-4}$alkyl or $CO_2C_{1-4}$alkyl with a compound of formula (III):

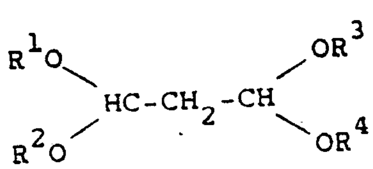

(III)

in which $R^1$ to $R^4$ are each $C_{1-6}$alkyl groups or $R^1$ and $R^2$ taken together and/or $R^3$ taken together are ethylene; or

b) reacting a compound of formula (IV):

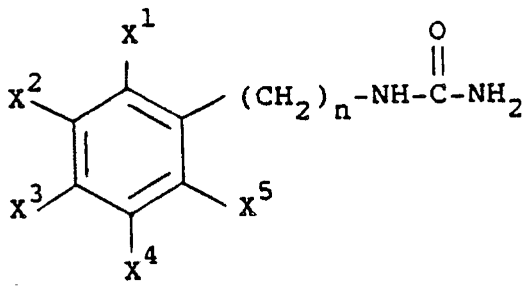

(IV)

in which n, $X^1$ to $X^5$ are as defined for compounds of the formula (II)

(1) with a compound of formula (III) as defined above in the presence of an acid to form a compound of formula (V):

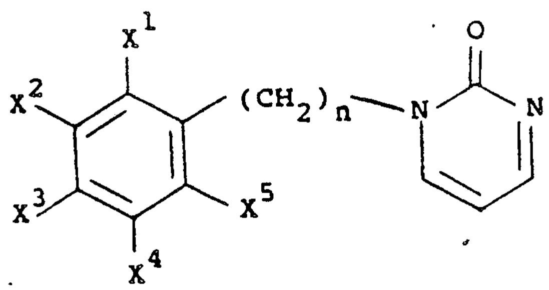

(V)

in which n and $X^1$ to $X^5$ are as defined for compounds of formula (II)

(2) followed by reaction with a compound of formula (VI) (Lawesson's reagent):

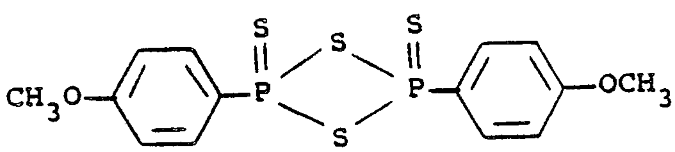

(VI)

to form a compound of formula (I) in which n and $X^1$ to $X^5$ are as defined for formula (II);

and optionally thereafter :

(1) converting a group $X^1$ to $X^5$ into a hydroxy, $CH_2OH$ or $CO_2H$ group and

(2) forming a pharmaceutically acceptable acid addition salt thereof.

2. A process according to claim 1 for preparing a compound in which n is 1.

3. A process according to claim 1 or 2 for preparing a compound in which one or two of $X^1$ to $X^5$ is halogen.

4. A process according to claim 1 or 2 for preparing a compound in which $X^4$ is halogen or $X^4$ and $X^2$ are halogen.

5. A process according to claim 1 for preparing a compound which is:

1-(3 fluorobenzyl)-1,2-dihydropyrimidine-2-thione;

1-(3,5-difluorobenzyl)-1,2 dihydropyrimidine-2-thione; or

1-benzyl-1,2-dihydropyrimidine 2-thione;

or a pharmaceutically acceptable acid addition salt thereof.

6. A process for preparing a pharmaceutical composition, which comprises combining a compound according to any one of claims 1 to 5 or a pharmaceutically acceptable acid addition salt thereof and a pharmaceutically acceptable carrier.

7. A process for preparing 1-(3-fluorobenzyl)-1,2-dihydropyrimidine-2-thione or a pharmaceutically acceptable acid addition salt thereof which comprises reacting N-(3-fluorobenzyl)thiourea with malondialdehyde tetramethyl acetal and thereafter optionally forming a pharmaceutically acceptable acid addition salt thereof.

8. A process for preparing 1-(3,5-difluorobenzyl)-1,2-dihydropyrimidine-2-thione or a pharmaceutically acceptable acid addition salt thereof which comprises reacting N-(3,5-difluorobenzyl)thiourea with malondialdehyde tetramethyl acetal and thereafter optionally forming a pharmaceutically acceptable acid addition salt thereof.

9. A process for preparing 1-benzyl-1,2-dihydropyrimidine-2-thione or a pharmaceutically acceptable acid addition salt thereof which comprises reacting N-benzylthiourea with malondialdehyde tetramethyl acetal and thereafter optionally forming a pharmaceutically acceptable acid addition salt thereof.